(19) Europäisches Patentamt
European Patent Office
Office européen des brevets

(11) Veröffentlichungsnummer: **0 075 135**
**B1**

# (12) EUROPÄISCHE PATENTSCHRIFT

(45) Veröffentlichungstag der Patentschrift: **11.09.85**

(51) Int. Cl.⁴: **A 61 K 31/635,** C 07 D 261/16 // (A61K31/635, 31:505)

(21) Anmeldenummer: **82107891.2**

(22) Anmeldetag: **27.08.82**

(54) **Verfahren zur Herstellung von Sulfonamid-Potentiator-Lösungen und ein neues Sulfanilamid.**

(30) Priorität: **23.09.81 CH 6140/81**

(43) Veröffentlichungstag der Anmeldung: **30.03.83 Patentblatt 83/13**

(45) Bekanntmachung des Hinweises auf die Patenterteilung: **11.09.85 Patentblatt 85/37**

(84) Benannte Vertragsstaaten: **AT BE CH DE FR GB IT LI LU NL SE**

(56) Entgegenhaltungen:
**EP - A - 0 037 501**
**CH - A - 247 344**
**US - A - 2 538 557**
**US - A - 2 538 558**

**CHEMICAL ABSTRACTS, Band 48, Nr. 2, 25. Januar 1954, Spalte 865b,c,d, Columbus, Ohio, USA R. MEIER et al.: "The curative effect of aldehyde derivatives of sulfonamide compounds"**

(73) Patentinhaber: **F. HOFFMANN-LA ROCHE & CO. Aktiengesellschaft, CH-4002 Basel (CH)**

(72) Erfinder: **Kompis, Ivan, Dr., Lettenhofstrasse 6, CH-4104 Oberwil (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

**EP 0 075 135 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von wäßrigen Lösungen potenzierter Sulfonamide.

Kombinationen von Sulfonamiden und Sulfonamid-Potentiatoren (letztere im folgenden der Einfachheit halber Potentiatoren genannt) werden in großem Umfang zur Behandlung von bakteriellen Infektionen in der Human- und Veterinärmedizin eingesetzt. Infolge der unterschiedlichen Löslichkeitscharakteristiken der Sulfonamide und der Potentiatoren und infolge des Umstandes, daß eine schwache Base (Potentiator) mit einer schwachen Säure (Sulfonamid) in nicht stöchiometrischen Mengen kombiniert werden muß, bereitet die Herstellung von pharmazeutisch anwendbaren Lösungen, z. B. Injektionslösungen, von Kombinationen dieser Stoffe Schwierigkeiten. Die bisher vorgeschlagenen Lösungen konnten nicht vollumfänglich befriedigen, sei es im Hinblick auf die Verträglichkeit der Lösungsmittel, besonders größerer Mengen organischer Lösungsmittel, sei es in bezug auf die Freisetzung der Wirkstoffe oder die Haltbarkeit der Präparate, oder auch im Hinblick auf deren Herstellungskosten. Diesem Mangel soll mit der vorliegenden Erfindung abgeholfen werden, indem pharmazeutische Präparate in wäßriger Form oder in einfacher Weise in wäßrige Form überführbare Präparate bereitgestellt werden, die eine befriedigende Verträglichkeit und Wirksamkeit besitzen, eine hohe Wirkstoffkonzentration und physiologisch günstige pH-Werte aufweisen, ausreichend stabil sind und keine kostspieligen Hilfsstoffe enthalten.

Es wurde gefunden, daß die erfindungsgemäß erhältlichen Lösungen in vitro die gleiche antibakterielle Wirkung entfalten, wie sie für die Kombination des entsprechenden Sulfonamids und des Potentiators zu erwarten war.

Gegenstand der vorliegenden Erfindung ist ein Verfahren zur Herstellung wäßriger Lösungen potenzierter Sulfonamide auf der Basis einer Verbindung der Formel

$$R^1-CH-(R)_2 \qquad (I)$$

worin $R^1$ Wasserstoff oder einen Rest $-CH_2OH$ oder $-CH(OH)CH_2OH$ und R den über die Aminogruppe gebundenen Rest eines antibakteriell wirksamen Sulfonamide bedeuten,
und einem Sulfonamid-Potentiator, und Trockenrückständen solcher Lösungen, dadurch gekennzeichnet, daß man eine Verbindung der Formel I, einen Sulfonamid-Potentiator und gegebenenfalls ein der Verbindung der Formel I entsprechendes Sulfonamidsalz oder ein der Verbindung der Formel I entsprechendes Sufonamid und die dem Sulfonamid äquivalente Menge Base in Wasser löst; oder daß man eine Verbindung der Formel I und einen Sulfonamid-Potentiator in einem inerten, aprotischen, organischen Lösungsmittel löst, danach das Lösungsmittel entfernt und den Rückstand in Wasser, das eine dem Sulfonamid äquimolare Menge Base enthält, löst; und gewünschtenfalls die Lösung zur Trockne bringt.

Die Verbindungen der Formel I können dadurch erhalten werden, daß man ein antibakteriell wirksames Sulfonamid in saurer Lösung mit einem Aldehyd $R^1-CHO$ umsetzt.

Beispiele von in den Verbindungen I in Form des Restes R enthaltenen antibakteriell wirksamen Sulfonamiden sind insbesondere $N^1$-heterocyclisch substituierte Sulfonamide, wie solche mit einem 5- oder 6gliedrigen Heterocyclus, wie einem Pyrimidin-, Pyrazin-, Pyridazin-, Oxazol-, Isoxazol-Ring. Spezifische Beispiele von Sulfonamiden sind Sulfadiazin, Sulfamethoxazol, Sulfatroxazol, Sulfamerazin, Sulfadoxin, Sulfadimethoxin, Sulfamethazin, Sulfachinoxalin, Sulfachlorpyridazin, Sulfaguanidin, Sulfalen, Sulfametin, Sulfamethoxin, Sulfamethoxypyridazin, Sulfamethylphenazol, Sulfaphenazol, Sulfamoxol, Sulfapyrazin, Sulfapyridazin, Sulfapyridin, Sulfasymazin und Sulfametrol.

Der Ausdruck »Potentiator« bezeichnet Verbindungen, die die antibakterielle Wirkung von Sulfonamiden mehr als additiv verstärken. Solche Sulfonamid-Potentiatoren sind insbesondere Verbindungen, welche die Dihydrofolat-Reduktase hemmen, wie vorzugsweise 2,4-Diaminopyrimidinderivate. Beispiele solcher 2,4-Diaminopyrimidinderivate sind im Phenylring substituierte 2,4-Diamino-5-benzylpyrimidine, wie

2,4-Diamino-5-(3,4,5-trimethoxybenzyl)-pyrimidin(Trimethoprim),
2,4-Diamino-5-[3,5-dimethoxy-4-2-methoxyäthoxy)benzyl]-pyrimidin (Tetroxoprim) und
2,4-Diamino-5-(3,5-dimethoxy-4-methylthiobenzyl)-pyrimidin (Metioprim).

Weitere Beispiele von Dihydrofolat-Reduktasehemmern sind

2,4-Diamino-5-(4-brom-3,5-dimethoxybenzyl)-pyrimidin;
2,4-Diamino-5-[3,5-diäthoxy-4-(pyrrol-1-yl)-benzyl]-pyrimidin;
2,4-Diamino-5-(3,5-dimethoxy-4-dimethylaminobenzyl)-pyrimidin;
2,4-Diamino-5-(3,4,dimethoxybenzyl)-pyrimidin (Diaveridin);
2,4-Diamino-5-(p-chlorphenyl)-6-äthylpyrimidin (Pyrimethamin) und
2,4-Diamino-5-(2-methyl-4,5-dimethoxybenzyl)-pyrimidin.

Der zur Herstellung der Verbindungen der Formel I verwendete Aldehyd $R^1CHO$ ist vorzugsweise Formaldehyd.

Die Umsetzung eines Sulfonamids mit dem Aldehyd $R^1CHO$ zur Herstellung einer Verbindung der Formel I wird zweckmäßig so vorgenommen, daß man das Sulfonamid unter Zusatz einer Säure in Wasser löst und die gegebenenfalls filtrierte Lösung mit wäßriger Aldehydlösung versetzt, wobei die Verbindung der Formel I ausfällt und z. B. durch Filtration der Reaktionslösung isoliert werden kann. Als Säure wird zweckmäßig eine Mineralsäure, wie Salzsäure verwendet. Die Säurekonzentration ist nicht in engerem Bereich kritisch, sie kann z. B. z. B. 0,1—2-normal, insbesondere 1-normal sein. Die Reaktion kann durch Vermischen der Reaktionspartner, zweckmäßig bei Raumtemperatur durchgeführt werden.

Von besonderem Interesse als Bestandteil der erfindungsgemäß erhältlichen Präparate sind die Kombinationen von Trimethoprim mit Verbindungen der Formel I, die sich von Sulfamethoxazol, Sulfadiazin, Sulfadoxin und Sulfametrol ableiten; ferner von Tetroxoprim und Sulfadiazinverbindungen der Formel I; oder von Pyrimethamin und Sulfadoxinverbindungen der Formel I.

Die erfindungsgemäße Herstellung der wäßrigen Lösungen kann durch Vermischen der Bestandteile und Erwärmen, zweckmäßig bis auf etwa 80° C, vorgenommen werden.

In einer anderen Ausführungsform der Erfindung löst man eine Verbindung der Formel I und einen Potentiator in einem inerten, aprotischen, organischen Lösungsmittel, wie z. B. Dioxan, vorzugsweise unter Erwärmen, z. B. auf bis etwa 80° C, und entfernt das Lösungsmittel, z. B. durch Abdampfen. Der so erhaltene, lösungsmittelfreie Rückstand kann in Wasser unter Zusatz einer dem anwesenden Sulfonamid äquivalenten Menge Base gelöst werden.

Der Anteil an Sulfonamid bzw. einer Verbindung der Formel I in den erfindungsgemäß erhältlichen Lösungen richtet sich nach der therapeutischen Wirksamkeit der Sulfonamid-Potentiator-Kombinationen. Bei den handelsüblichen Kombinationen ist das Molverhältnis Sulfonamid: Potentiator $\geqq 1:1$, im Falle der handelsüblichen Kombinationen Sulfamethoxazol : Trimethoprim ist es z. B. etwa 5,7 : 1 (entsprechend einem Gewichtsverhältnis von 5 : 1). Zur Herstellung einer eine solche Kombination enthaltenden Lösung werden zweckmäßig pro Mol Potentiator $\geqq$0,5 Mol, vorzugsweise 1,5 bis 2,5 Mol Verbindung der Formel I eingesetzt. Erwünschtenfalls können der Lösung weitere Mengen Sulfonamid zugesetzt werden, wobei man das Sulfonamid in Form eines Salzes verwendet oder der Lösung eine dem zusätzlich zugegebenen Sulfonamid äquivalente Menge Base zusetzt. Als Basen für die Salzbildung kommen insbesondere Alkalihydroxide, wie Natrium- oder Kaliumhydroxid, oder pharmazeutisch verwendbare organische Basen, wie N-Methylglucamin, oder basische Aminosäuren, wie Lysin, Arginin oder Ornithin in Betracht.

Die Konzentration der so hergestellten Lösungen, d. h., der Gehalt an gelösten Stoffen, kann 40 Gew.-% oder mehr betragen, vorzugsweise stellt man 10—20%ige Lösungen her. Bei bestimmten Sulfonamid-Potentiator-Kombinationen kann es bei der Herstellung zweckmäßig sein, der Lösung zusätzlich ein geeignetes, mit Wasser mischbares organisches Lösungsmittel, wie Glycofurol oder Polyäthylenglykol 400, zuzusetzen.

Die Lösungen können mittels an sich bekannter galenischer Techniken zur Trockne gebracht werden, z. B. durch Gefrier- oder Sprühtrocknung. Die so erhaltenen Trockenpräparate, die ebenfalls Gegenstand der Erfindung sind, können, gegebenenfalls nach vorheriger Sterilisation, durch Zusatz von Wasser wieder in Lösungen, z. B. Injektionslösungen, übergeführt werden.

Die Lösungen können weiterhin mittels in der Galenik bei der Herstellung parenteraler Applikationsformen üblichen Techniken sterilisiert werden, z. B. durch Hitzesterilisation oder Sterilfiltration.

Die Verwendungsmöglichkeit der erfindungsgemäßen Verbindungen der Formel I beschränkt sich nicht auf die Herstellung von Injektionslösungen; sie können auch für andere Zwecke, bei denen eine Sulfonamid-Potentiator-Kombination in gelöster Form eingesetzt werden soll, verwendet werden.

Die aus Verbindungen der Formel I und Sulfonamid-Potentiatoren hergestellten Präparate zeigen in vitro und in vivo die bekannte antibakterielle Aktivität der entsprechenden Sulfonamid-Potentiator-Kombinationen und können daher für die gleichen Indikationen Verwendung finden.

Die nachfolgenden Beispiele erläutern die Erfindung weiter. Die Temperaturen sind in Grad Celsius angegeben.

Wäßrige Lösungen auf der Basis eines wasserlöslichen Sulfonamid-Salzes, eines Sulfonamid-Potentiators und von Formaldehyd, Glykolaldehyd oder Glycerinaldehyd und Trockenrückstände solcher Lösungen werden in der nicht vorveröffentlichten europäischen Patentanmeldung 0 037 501 beansprucht.

## Beispiel 1

38 g Sulfamethoxazol wurden bei 25° in einer Mischung von 150 ml konz. HCl und 1350 ml Wasser unter Rühren fast vollständig gelöst, sofort filtriert und unter Rühren mit 19,2 ml ca. 35%iger Formaldehyd-Lösung versetzt. Die entstandene Suspension wurde für weitere 10 Minuten gerührt, das feste Produkt abgenutscht und dreimal mit je 150 ml Wasser gewaschen. Nach Trocknung wurden 27 g von $N^4,N^{4'}$-Methylen-bis[$N^1$-(5-methyl-3-isoxazolyl)sulfanilamid], Smp. 180—188° erhalten.

Beispiel 2

Zu einer Lösung von 570 mg $N^4,N^{4'}$-Methylen-bis[$N^1$-(5-methyl-3-isoxazolyl)sulfanilamid] und 242 mg Sulfamethoxazol in 3,15 ml NaOH und 5 ml Wasser wurden 100 mg Trimethoprim gegeben. Die Suspension wurde für 10 Minuten auf 80° erwärmt. Nach Abkühlen auf Raumtemperatur wurde das Gesamtvolumen auf 10 ml Wasser aufgefüllt. Diese Lösung kann bei −32° lyophilisiert werden. Das Lyophilisat kann in 5 ml Wasser zu Injektionszwecken aufgelöst werden.

Beispiel 3

Zu einer Lösung von 0,8 g Trimethoprim in 150 ml Dioxan wurden bei Raumtemperatur sukzessiv eine Lösung von 2,86 g $N^4,N^{4'}$-Methylen-bis[$N^1$-(5-methyl-3-isoxazolyl)-sulfanilamid] in 150 ml Dioxan und 1,22 g Sulfamethoxazol in 150 ml Dioxan gegeben. Die leicht trübe Lösung wurde 1 Stunde bei 85° erwärmt, auf 25° abgekühlt, filtriert und das Lösungsmittel unter vermindertem Druck entfernt. Der feste Rückstand wurde in 15,75 ml 1N NaOH gelöst und das Gesamtvolumen auf 25 ml mit Wasser aufgefüllt. Diese Lösung kann bei −32° lyophilisiert werden. Das Lyophilisat kann in 25 ml Wasser zu Injektionszwecken aufgelöst werden.

**Patentansprüche für die Vertragsstaaten: BE, CH, DE, FR, GB, IT, LI, LU, NL und SE**

1. Verfahren zur Herstellung wäßriger Lösungen potenzierter Sulfonamide auf der Basis einer Verbindung der Formel

$$R^1-CH-(R)_2 \qquad (I)$$

worin $R^1$ Wasserstoff oder einen Rest $-CH_2OH$ oder $-CH(OH)CH_2OH$ und R den über die Aminogruppe gebundenen Rest eines antibakteriell wirksamen Sulfonamids bedeuten, und einem Sulfonamid-Potentiator, und Trockenrückständen solcher Lösungen, dadurch gekennzeichnet, daß man eine Verbindung der Formel I, einen Sulfonamid-Potentiator und gegebenenfalls ein der Verbindung der Formel I entsprechendes Sufonamidsalz oder ein der Verbindung der Formel I entsprechendes Sulfonamid und die dem Sulfonamid äquivalente Menge Base in Wasser löst; oder daß man eine Verbindung der Formel I und einen Sulfonamid-Potentiator in einem inerten, aprotischen, organischen Lösungsmittel löst, danach das Lösungsmittel entfernt und den Rückstand in Wasser, das eine dem Sulfonamid äquimolare Menge Base enthält, löst; und gewünschtenfalls die Lösung zur Trockene bringt.

2. Verfahren nach Anspruch 1, dadurch gekennzeichnet, daß in der Verbindung der Formel I $R^1$ Wasserstoff ist.

3. Verfahren nach den Ansprüchen 1 oder 2, dadurch gekennzeichnet, daß in der Verbindung der Formel I R der über die Aminogruppe gebundene Rest von Sulfamethoxazol, Sulfadiazin, Sulfadoxin oder Sulfametrol ist.

4. Verfahren nach den Ansprüchen 1 oder 2, dadurch gekennzeichnet, daß in der Verbindung der Formel I R der über die Aminogruppe gebundene Rest von Sulfamethoxazol ist.

5. Verfahren nach den Ansprüchen 1—4, dadurch gekennzeichnet, daß der Potentiator Trimethoprim ist.

6. $N^4,N^{4'}$-Methylen-bis[$N^1$-(5-methyl-3-isoxazolyl)-sulfanilamid].

**Patentansprüche für den Vertragsstaat: AT**

1. Verfahren zur Herstellung wäßriger Lösungen potenzierter Sulfonamide auf der Basis einer Verbindung der Formel

$$R^1-CH-(R)_2 \qquad (I)$$

worin $R^1$ Wasserstoff oder einen Rest $-CH_2OH$ oder $-CH(OH)CH_2OH$ und R den über die Aminogruppe gebundenen Rest eines antibakteriell wirksamen Sulfonamids bedeuten, und einem Sulfonamid-Potentiator, und Trockenrückständen solcher Lösungen, dadurch gekennzeichnet, daß man eine Verbindung der Formel I, einen Sulfonamid-Potentiator und gegebenenfalls ein der Verbindung der Formel I entsprechendes Sufonamidsalz oder ein der Verbindung der Formel I entsprechendes Sulfonamid und die dem Sulfonamid äquivalente Menge Base in Wasser löst; oder daß man eine Verbindung der Formel I und einen Sulfonamid-Potentiator in einem inerten, aprotischen, organischen Lösungsmittel löst, danach das Lösungsmittel entfernt und den Rückstand in Wasser, das eine dem Sulfonamid äquimolare Menge Base enthält, löst; und gewünschtenfalls die Lösung zur

Trockene bringt.

2. Verfahren nach Anspruch 1, dadurch gekennzeichnet, daß in der Verbindung der Formel I $R^1$ Wasserstoff ist.

3. Verfahren nach den Ansprüchen 1 oder 2, dadurch gekennzeichnet, daß in der Verbindung der Formel I R der über die Aminogruppe gebundene Rest von Sulfamethoxazol, Sulfadiazin, Sulfadoxin oder Sulfametrol ist.

4. Verfahren nach den Ansprüchen 1 oder 2, dadurch gekennzeichnet, daß in der Verbindung der Formel I R der über die Aminogruppe gebundene Rest von Sulfamethoxazol ist.

5. Verfahren nach den Ansprüchen 1—4, dadurch gekennzeichnet, daß der Potentiator Trimethoprim ist.

**Claims for the Contracting States: BE, CH, DE, FR, GB, IT, LI, LU, NL and SE**

1. A process for the manufacture of aqueous solutions of potentiated sulphonamides based on a compound of the formula

$$R^1-CH-(R)_2 \qquad (I)$$

wherein $R^1$ signifies hydrogen or a residue $-CH_2OH$ or $-CH(OH)CH_2OH$ and R signifies the residue of an antibacterially-active sulphonamide bonded via the amino group.
and a sulphonamide potentiator, and dry residues of such solutions, characterized by dissolving a compound of formula I, a sulphonamide potentiator and, if desired, a sulphonamide salt corresponding to the compound of formula I or a sulphonamide corresponding to the compound of formula I and the amount of base equivalent to the sulphonamide in water; or dissolving a compound of formula I and a sulphonamide potentiator in an inert, aprotic, organic solvent, thereafter removing the solvent and dissolving the residue in water which contains an amount of base equimolar to the sulphonamide: and, if desired, dyring the solution.

2. A process according to claim 1, characterized in that $R^1$ in the compound of formula I is hydrogen.

3. A process according to claims 1 or 2, characterized in that R in the compound of formula I is the residue of sulphamethoxazole, sulphadiazine, sulphadoxine or sulphametrole bonded via the amino group.

4. A process according to claims 1 or 2, characterized in that R in the compound of formula I is the residue of sulphamethoxazole bonded via the amino group.

5. A process according to claims 1—4, characterized in that the potentiator is trimethoprim.

6. $N^4,N^{4'}$-Methylene-bis[$N^1$-(5-methyl-3-isoxazolyl)sulphanilamide].

**Claims for the Contracting State: AT**

1. A process for the manufacture of aqueous solutions of potentiated sulphonamides based on a compound of the formula

$$R^1-CH-(R)_2 \qquad (I)$$

wherein $R^1$ signifies hydrogen or a residue $-CH_2OH$ or $-CH(OH)CH_2OH$ and R signifies the residue of an antibacterially-active sulphonamide bonded via the amino group.
and a sulphonamide potentiator, and dry residues of such solutions, characterized by dissolving a compound of formula I, a sulphonamide potentiator and, if desired, a sulphonamide salt corresponding to the compound of formula I or a sulphonamide corresponding to the compound of formula I and the amount of base equivalent to the sulphonamide in water; or dissolving a compound of formula I and a sulphonamide potentiator in an inert, aprotic, organic solvent, thereafter removing the solvent and dissolving the residue in water which contains an amount of base equimolar to the sulphonamide: and, if desired, dryring the solution.

2. A process according to claim 1, characterized in that $R^1$ in the compound of formula I is hydrogen.

3. A process according to claims 1 or 2, characterized in that R in the compound of formula I is the residue of sulphamethoxazole, sulphadiazine, sulphadoxine or sulphametrole bonded via the amino group.

4. A process according to claims 1 or 2, characterized in that R in the compound of formula I is the residue of sulphamethoxazole bonded via the amino group.

5. A process according to claims 1—4, characterized in that the potentiator is trimethoprim.

**Revendications pour les Etats contractants: BE, CH, DE, FR, GB, IT, LI, LU, NL, SE**

1. Procédé de préparation de solutions aqueuse de sulfonamides potentialisées à base d'un composé de formule

$$R1-CH-(R)_2 \quad \text{(I)}$$

où R1 représente un hydrogène ou un radical $-CH_2OH$ ou $-CH(OH)CH_2OH$ et R un radical d'un sulfonamide à action antibactérienne lié par l'intermédiaire du groupe amino,
et d'un potentialisateur de sulfonamide, et de résidus secs de telles solutions, caractérisé en ce qu'on dissout dans l'eau un composé de formule I, un potentialisateur de sulfonamide et éventuellement un sel de sulfonamide correspondant au composé de formule I ou un sulfonamide correspondant au composé de formule I et la quantité de base équivalant au sulfonamide: ou en ce qu'on dissout un composé de formule I et un potentialisateur de sulfonamide dans un solvant organique aprotique inerte, puis en ce qu'on retire le solvant et en ce qu'on dissout le résidu dans de l'eau qui contient une quantité de base équimolaire par rapport à celle du sulfonamide: et si on le désire en ce qu'on dessèche la solution.

2. Procédé selon la revendication 1, caractérisé en ce que dans le composé de formule I R1 est un hydrogène.

3. Procédé selon les revendications 1 et 2, caractérisé en ce que dans le composé de formule I R est le radical du sulfaméthoxazole, de la sulfadiazine, de la sulfadoxine ou du sulfamétrol lié par l'intermédiaire du groupe amino.

4. Procédé selon les revendications 1 et 2, caractérisé en ce que dans le composé de formule I R est le radical du sulfaméthoxazole lié par l'intermédiaire du groupe amino.

5. Procédé selon les revendications 1-4, caractérisé en ce que le potentialisateur est le triméthoprime.

6. $N^4$,$N^{4'}$-méthylène-bis[$N^1$-(5-méthyl-3-isoxazolyl)-sulfanilamide].

**Revendications pour l'Etat contractant: AT**

1. Procédé de préparation de solutions aqueuses de sulfonamides potentialisées à base d'un composé de formule

$$R1-CH-(R)_2 \quad \text{(I)}$$

où R1 représente un hydrogène ou un radical $-CH_2OH$ ou $-CH(OH)CH_2OH$ et R un radical d'un sulfonamide à action antibactérienne lié par l'intermédiaire du groupe amino,
et d'un potentialisateur de sulfonamide, et de résidus secs de telles solutions, caractérisé en ce qu'on dissout dans l'eau un composé de formule I, un potentialisateur de sulfonamide et éventuellement un sel de sulfonamide correspondant au composé de formule I ou un sulfonamide correspondant au composé de formule I et la quantité de base équivalant au sulfonamide: ou en ce qu'on dissout un composé de formule I et un potentialisateur de sulfonamide dans un solvant organique aprotique inerte, puis en ce qu'on retire le solvant et en ce qu'on dissout le résidu dans de l'eau qui contient une quantité de base équimolaire par rapport à celle du sulfonamide: et si on le désire en ce qu'on dessèche la solution.

2. Procédé selon la revendication 1, caractérisé en ce que dans le composé de formule I R1 est un hydrogène.

3. Procédé selon les revendications 1 et 2, caractérisé en ce que dans le composé de formule I R est le radical du sulfaméthoxazole, de la sulfadiazine, de la sulfadoxine ou du sulfamétrol lié par l'intermédiaire du groupe amino.

4. Procédé selon les revendications 1 et 2, caractérisé en ce que dans le composé de formule I R est le radical du sulfaméthoxazole lié par l'intermédiaire du groupe amino.

5. Procédé selon les revendications 1-4, caractérisé en ce que le potentialisateur est le triméthoprime.